# EUROPÄISCHE PATENTSCHRIFT

(11) **EP 1 294 388 B1**
(45) Veröffentlichungstag und Bekanntmachung des Hinweises auf die Patenterteilung: **07.01.2004**
(21) Anmeldenummer: 01953136.7
(22) Anmeldetag: 29.06.2001
(51) Int. Cl.: A61K 35/78, A61P 35/00, A61P 5/30, A61P 19/10, A61P 25/28, A61P 9/00, A61P 13/08

(54) **EXTRAKTE AUS SOPHORA-ARTEN, VERFAHREN ZU IHRER HERSTELLUNG UND VERWENDUNG**
EXTRACTS FROM SOPHORA SPECIES, METHOD FOR PRODUCING THE SAME AND THEIR USE
EXTRAITS D' ESPECES DE SOPHORA, LEUR PROCEDE DE PRODUCTION ET LEUR UTILISATION

(30) Priorität: 29.06.2000 DE 10031651
(43) Veröffentlichungstag der Anmeldung: 26.03.2003
(73) Patentinhaber: Dr. Willmar Schwabe GmbH & Co. KG, 76227 Karlsruhe (DE)
(72) Erfinder: ERDELMEIER, Clemens, 76139 Karlsruhe (DE); JAGGY, Hermann, 76229 Karlsruhe (DE); KOCH, Egon, 76229 Karlsruhe (DE)
(74) Vertreter: Adam, Holger, Dr.
(86) Internationale Anmeldenummer: PCT/DE2001/002460
(87) Internationale Veröffentlichungsnummer: WO 2002/000236

(56) Entgegenhaltungen:
- EP-A- 0 998 924
- SHI YONG RYU ET AL.: "IN VITRO ANTITUMOUR ACTIVITY OF FLAVONOIDS FROM SOPHORA FLAVESCENS" PHYTOTHERAPY RESEARCH., Bd. 11, Nr. 1, 1997, Seiten 51-53, XP001041444 JOHN WILEY & SONS LTD. CHICHESTER., GB ISSN: 0951-418X
- DATABASE BIOSIS [Online] BIOSCIENCES INFORMATION SERVICE, PHILADELPHIA, PA, US; Oktober 2000 (2000-10) KO W G ET AL: "Lavandulylflavonoids: A new class of in vitro apoptogenic agents from Sophora flavescens." Database accession no. PREV200000462363 XP002183326 & TOXICOLOGY IN VITRO, Bd. 14, Nr. 5, Oktober 2000 (2000-10), Seiten 429-433, ISSN: 0887-2333
- PATENT ABSTRACTS OF JAPAN vol. 014, no. 254 (C-0724), 31. Mai 1990 (1990-05-31) & JP 02 073079 A (DAICEL CHEM IND LTD), 13. März 1990 (1990-03-13)

## Beschreibung

Die vorliegende Erfindung betrifft Extrakte aus *Sophora*-Arten, Verfahren zu ihrer Herstellung und ihre Verwendung zur Herstellung eines Medikaments zur Prophylaxe und Therapie von Krankheitszuständen, die durch einen Mangel an Östrogenen oder durch eine Dysregulation des Geschlechtshormonstoffwechsels, insbesondere Östrogenstoffwechsels verursacht werden.

Unregelmäßige Menstruationszyklen treten bei Frauen ab einem Alter von etwa 40 Jahren auf und markieren den Beginn der Menopause. Diese Phase von Veränderungen im endokrinen System, Klimakterium genannt, kann eine Dekade und länger anhalten. Sie resultiert aus einer Erschöpfung der Follikelbildung und deren verminderte Reaktion auf Gonadotropin. Als Folge des Follikelausfalls sinkt die Produktion von Östrogen und bleibt schließlich völlig aus. Damit verbunden können sich eine Reihe von Symptomen entwickeln, z.B. Hitzewallungen, Depressionen, Angstzustände, geistige Verwirrtheit, Schlaflosigkeit usw. Außerdem können als Folge des Absinkens der Östrogenproduktion ernsthafte Gesundheitsprobleme wie Osteoporose, Herzinsuffizienz, Schlaganfall und Krebs auftreten.

Obwohl eine Hormonersatztherapie mit Östrogenen zur Erleichterung klimakterischer Beschwerden sehr wirksam ist, gibt es zahlreiche Studien, die zeigen, dass damit ein erhöhtes Risiko für Brust- und Gebärmutterkrebs, Herz-Kreislauf-Erkrankungen und Veränderungen des Lebermetabolismus verbunden sein kann. Durch die Gabe von Progestinen kann das Krebsrisiko vermindert werden. Der protektive Effekt der Progestine scheint jedoch bei einer Langzeitanwendung zu verschwinden und es wird vermutet, dass die Hormonersatztherapie (HRT) mit einem bis zu 35-40 % erhöhten Brustkrebsrisiko verbunden ist. Wegen der Nebenwirkungen und deutlicher Zweifel an der Zuverlässigkeit einer solchen Therapie überhaupt, steht die Medizin der HRT bei Frauen in der Postmenopause eher ablehnend gegenüber. Es wird geschätzt, dass nur ca. 20 % der USamerikanischen Frauen in der Postmenopause eine HRT erhalten, wobei lediglich 40 % dieser Frauen die Therapie über mehr als ein Jahr fortsetzen.

Substanzen, welche bei Mensch oder Tier östrogenagonistisch wirken oder mit Östrogen interagieren, wurden in vielen Pflanzen gefunden. Mindestens 20 verschiedene Stoffgruppen mit östrogenen/antiöstrogenen Eigenschaften aus mehr als 300 Pflanzen von mehr als 16 Pflanzenfamilien wurden bis jetzt gefunden. Die meisten bekannten Phytoöstrogene gehören zu den Gruppen der Isoflavone, Lignane oder Cumestane. Erst kürzlich wurde ein weiteres, hochpotentes Phytoöstrogen identifiziert, das 8-Prenylnaringenin (S.R. Milligan et al., J. Clin. Endocrinol. Metab. 84, 2249-2252 (1999)). Die östrogene Potenz von 8-Prenylnaringenin in vitro wurde anhand der Stimulation der alkalischen Phosphatase in Ishikawa-Var-I-Zellen getestet. Dabei fand man, dass 8-Prenylnaringenin wesentlich aktiver war als bislang bekannte Phytoöstrogene wie Coumestrol, Genistein oder Daidzein, und nur wenig schwächer wirkte als 17β-Östradiol.

Epidemiologische und kontrollierte Studien an Populationen in den USA, Europa, China und Japan haben gezeigt, daß ein enge negative Korrelation zwischen der Aufnahme von Phytoöstrogenen mit der Nahrung und dem Auftreten verschiedener Tumoren, insbesondere Brustkrebs und Prostatakarzinom, besteht. Hemmende Wirkungen von Phytoöstrogene auf das Wachstum von Mamma- und Prostatatumorzellen wurde auch in tierexperimentellen Untersuchungen bestätigt. Die präventiven Eigenschaften von Phytoöstrogenen beruhen offenbar auf einer Vielzahl von unterschiedlichen biologischen Eigenschaften, die diese Verbindungen von synthetischen und natürlichen Östrogenen unterscheiden. Abhängig von der Dosis und dem endogenen Hormonstatus verfügen Phytoöstrogene über östrogene oder auch antiöstrogene Wirkungen. Andere biologische Effekte beinhalten z.B. die Hemmung von Tyrosinkinasen, DNA Topoisomerasen, ribosomale S6Kinase, Ornithindecaroxylase, Aromatase oder 5α-Reduktase. Außerdem verfügen Phytoöstrogene häufig über antioxidative Eigenschaften und es ist offensichtlich die Summe dieser unterschiedlichen Aktivitäten, die zur Hemmung der Tumorentstehung und des Tumorwachstums beiträgt (Tham et al., J. Clin. Endocrinol. Metab. 83, 2223-2235 (1998)).

Der vorliegenden Erfindung liegt daher die Aufgabe zugrunde, Pflanzenextrakte bereitzustellen, die zur Herstellung eines Medikaments zur Prophylaxe und Therapie von Krankheitszuständen geeignet sind, die durch einen Mangel an Östrogenen oder durch eine Dysregulation des Geschlechtshormonstoffwechsels, insbesondere Östrogenstoffwechsels verursacht werden.

Eine weitere Aufgabe der vorliegenden Erfindung ist die Bereitstellung von Verfahren zur Herstellung dieser Extrakte.

Diese Aufgaben werden erfindungsgemäß durch die Extrakte aus Soph ora-Arten gemäß den Patentansprüchen 1- 5, dem Verfahren zu ihrer Herstellung gemäß den Patentansprüchen 6-16, die pharmazeutische Zubereitung gemäß Patentanspruch 17, sowie die Verwendung der Extrakte gemäß den Patentansprüchen 18-20 gelöst.

Die traditionelle chinesische Medizin kennt einige *Sophora*-Arten (Fabaceae), deren Wurzeln arzneilich verwendet werden. Diese sind *Sophora flavescens, S. subprostrata, S. alopeculoides, S. japonica, S. tonkinensis, S. tomentosa, S. moorcroftiana* und *S. leachiana.* Von diesen kommt *Sophora flavescens* größere Bedeutung zu. Sie ist deshalb Bestandteil des chinesischen Arzneibuchs (Sophorae falvescentis radix, "Kushen", Arzneibuch der chinesischen Medizin 9/1997) und wird traditionell zur Behandlung von Durchfall, gastrointestinalen Hämorrhagien und Ekzemen medizinisch verwendet (W. Tang, G. Eisenbrand, Chinese Drugs of Plant Origin, Springer Verlag, Berlin, 1992).

Die Wurzeln von *S. flavescens*, aber auch anderer *Sophora*-Arten enthalten eine Reihe von Chinolizidin-Alkaloiden als Hauptinhaltsstoffe. Das chinesische Arzneibuch schreibt vor, dass der titrimetrische Gesamtgehalt an Alkaloiden 2 % nicht unterschreiten darf. Hauptalkaloide sind Matrin und Oxymatrin.

Neben den Alkaloiden wurde auch eine Vielzahl von Flavonen und verwandten Verbindungen und Triterpensaponine in den Wurzeln von *S. flavescens* sowie etlichen anderen *Sophora*-Arten gefunden.

Verschiedene pharmakologische Eigenschaften sind für *Sophora flavescens* und einzelne Inhaltsstoffe bzw. Inhaltsstoffgruppen beschrieben worden. Insbesondere die Alkaloide werden für antiarrhythmische, antiasthmatische und antitussive Effekte sowie für Antiulcus-Effekte verantwortlich gemacht (Tang und Eisenbrand, s.o.). Auch antineoplastische Effekte und immunsuppressive Eigenschaften wurden beobachtet.

M. Kuroyanagi et al. Journal of Natural Products 1999, 62(12), 1595-1599, berichten über antibakterielle und antiandrogene Flavonoide aus *Sophora flavescens*. Es wurde gefunden, dass nahezu alle aus *Sophora flavescens* isolierten Prenylflavon-Derivate eine antiandrogene Wirkung aufwiesen.

W.M. Mazur et al., Nutritional Biochemistry 1998, 9, 193-200, berichten über die quantitative Bestimmung der Isoflavone Formononetin, Biochanin A, Daidzein, Genistein und Coumestrol und der Lignane Secoisolariciresinol und Matairesinol in Leguminosensamen, wobei u.a. auch Samen von *Sophora japonica* untersucht worden sind.

*S. flavescens* und *S. japonica* werden in der Toxikologie als toxisch eingestuft. Als Gifte werden vor allem Alkaloide vom Matrin-Typ vermutet (T. Blaszczyk, Deutsche Apoth. Ztg. 2001, 141 (14), 1687-1696). In klinischen Prüfungen wurden toxische Effekte wie heftiges Herzklopfen, Atemnot und Krämpfe für die *Sophora*-Alkaloide beobachtet. In Tierversuchen konnten toxische Effekte für Oxymatrin nachgewiesen werden (K.-C. Huang, The Pharmacology of Chinese Herbs, CRC Press, Boca Raton, 1993).

Die EP 0 998 924 A1 beschreibt ein Antitumormittel, das aus Pflanzen erhältliche Verbindungen mit Topoisomerase II inhibitorischer Aktivität enthält. So wird u.a. im Beispiel 8 die Gewinnung von Leachianol A aus *Sophora* beschrieben. Hierbei wird ein Trockenpulver aus *Sophora* einer sequentiellen Extraktion mit Aceton und Methanol unter Rückfluss unterworfen. Der Aceton-Extrakt wird unter vermindertem Druck konzentriert und mit Wasser versetzt. Das resultierende Gemisch wird einer schrittweisen Verteilungsextraktion unter Verwendung von Benzol, Ethylacetat und n-Butanol unterworfen. Der Ethylacetat-Extrakt wird unter vermindertem Druck konzentriert und dabei ein Rückstand erhalten. Der so erhaltene Rückstand wird durch Kieselgelchromatographie unter Verwendung eines Benzol-Aceton-Lösungsmittelgemisches gereinigt, wobei Leachianol A erhalten wird. Der in diesem Beispiel 8 beschriebene Ethylacetat-Extrakt unterscheidet sich von den erfindungsgemäßen Extrakten dadurch, dass die erfindungsgemäßen Extrakte Maacckiain enthalten und Maackiainglucosid stark angereichert (ca. fünffach) ist. Bei diesen beiden Verbindungen handelt es sich um wirksamkeitsrelevante Inhaltsstoffe des erfindungsgemäßen Extraktes wie anhand eines Östrogenrezeptorbindungsassays entsprechend dem Beispiel 5 von den Erfindern erstmals gezeigt werden konnte. Die EP 0 998 924 A1 gibt keinen Hinweis auf die Verwendung von Extrakten aus *Sophora*-Arten zur Prophylaxe und Therapie von Krankheitszuständen, die durch einen Mangel an Östrogenen oder durch eine Dysregulation des Geschlechtshormonstoffwechsels, insbesondere Östrogenstoffwechsels verursacht werden.

Im Zusammenhang mit der vorliegenden Erfindung besonders bevorzugte *Sophora*-Arten sind *Sophora flavescens* und *Sophora subprostrata*, für deren wässrig-alkoholische Extrakte aus den Wurzeln überraschenderweise im Rahmen der vorliegenden Erfindung starke östrogene Wirkungen festgestellt wurden. Diese wässrig-alkoholischen Extrakte konnten durch weitere Trennung und Aufreinigung angereichert werden. Es zeigte sich, dass die beobachteten pharmakologischen Effekte durch das Zusammenspiel der in den Extrakten vorliegenden Isoflavone wie Genistein und Daidzein, Flavone wie 8-Prenylnaringenin, Kushenol X, 8-Prenylkämpferol, Leachianon G und Kushenol E, Chalkone und Pterocarpane wie Maackiain und Maackiainglucosid.sowie noch nicht identifizierte weitere flavonoidartige Verbindungen verursacht werden. In diesem Zusammenhang ist auch das von den Erfindern neu gefundene Chalkon 2,4,4',6'-Tetrahydroxy-3'-lavandulyl-2'-Methoxychalcon der nachstehenden Formel 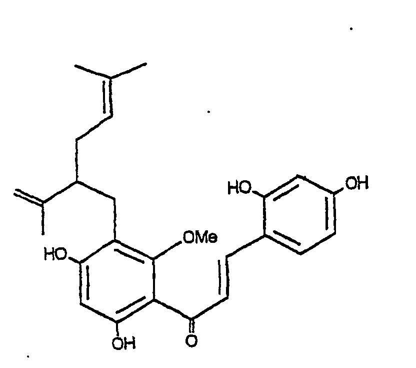 zu nennen.

Figur 1 beschreibt die östrogene Aktivität von 70 % (v/v) (62 % (w/w)) Ethanol-Extrakten (gemäß der Beispiele 1a) und 2a)) aus *Sophora flavescens* und *Sophora subprostrata* in einem Hefe-Assay, verglichen mit 17β-Östradiol.

Figur 2 beschreibt die östrogene Aktivität von erfindungsgemäßen Extrakten (70 % (v/v) bzw. 62 % (w/w) Ethanol-Extrakte nach Verteilung mit Ethylacetat; gemäß der Beispiele 1b) und 2b)) aus *Sophora flavescens* und *Sophora subprostrata* in einem Hefe-Assay, verglichen mit 17β-Östradiol.

Figur 3 beschreibt die östrogene Aktivität von erfindungsgemäßen Extrakten (60 % (w/w) Ethanol-Extrakte nach Verteilung mit Ethylacetat gemäß Beispiel 3) aus *Sophora flavescens* in einem Hefe-Assay, verglichen mit 17β-Östradiol.

Entsprechende Extrakte können allgemein durch Extraktion mit einem Lösungsmittel mittlerer Polarität, das aus der Gruppe bestehend aus wässrigen Alkoholen, wässrigen Ketonen und Estern einschließlich wässrigen oder wassergesättigten Estern ausgewählt ist, Abziehen des Lösungsmittels und nachfolgende Flüssig-Flüssig-Verteilung zwischen einem organischen Lösungsmittel und Wasser erhalten werden.

Erfindungsgemäß wird ein Extrakt aus *Sophora*-Arten bereitgestellt, erhältlich durch:
a) ein- oder mehrmaliges Extrahieren von *Sophora*-Arten mit einem Lösungsmittel ausgewählt aus der Gruppe bestehend aus
   i) wässrigen Alkoholen und wässrigen Ketonen; und
   ii) Estern einschließlich wässrigen oder wassergesättigten Estern;
b) Abziehen des Lösungsmittels von der in Schritt a)i) erhaltenen alkoholischen oder ketonischen Extraktionslösung;
c)i) ein- oder mehrmalige Verteilung des in Schritt b) erhaltenen Rückstands zwischen einem organischen Lösungsmittel ausgewählt aus der Gruppe bestehend aus Ethylacetat, tert-Butylmethylether, n-Butanol und Butanon und Wasser und Vereinigen der erhaltenen Lösungsmittelphasen; oder
c)ii) ein- oder mehrmalige direkte Verteilung des in Schritt a)ii) erhaltenen Ethylacetatextrakts mit Wasser; und
d) Einengen der in c) erhaltenen vereinigten organischen Lösungsmittelphasen zur Trockne.

Das Verhältnis Droge zu Lösungsmittel liegt bei jedem Extrahieren im Bereich von etwa 1:7 bis etwa 1:10.

In einer bevorzugten Ausführungsform wird die Extraktion in Schritt a) zweimal ausgeführt.

Das in Schritt a) verwendete Lösungsmittel ist vorzugsweise ausgewählt aus der Gruppe bestehend aus wässrigem Ethanol, wässrigem Aceton und wässrigem oder wassergesättigtem Ethylacetat. Insbesondere ist das in Schritt a) verwendete Lösungsmittel 70 % (v/v) (62 % (w/w)) Ethanol, 60 % (w/w) Ethanol oder wassergesättigtes Ethylacetat.

Die in Schritt a)i) verwendeten Alkohole bzw. Ketone werden vorzugsweise als 10-96 % (v/v) oder (w/w) , bzw. 10-99% (v/v) oder (w/w), insbesondere 50-92 % (v/v) oder (w/w) wässrige Gemische eingesetzt.

In Schritt c)i) ist Ethylacetat als Lösungsmittel bevorzugt.

Im Verfahrensschritt c) erfolgt im Wesentlichen eine Abreicherung des Gesamtalkaloidgehalts. Der erfindungsgemäß erhältliche Extrakt ist dadurch gekennzeichnet, dass der Gesamtalkaloidgehalt unterhalb 0,2 %, vorzugsweise unterhalb 0,1 % liegt. Insbesondere ist der erfindungsgemäße Extrakt alkaloidfrei. Alkaloidfrei bedeutet in diesem Zusammenhang, dass Alkaloidverbindungen mit den gängigen analytischen Verfahren wie HPLC nicht nachweisbar sind. Weiterhin ist der erfindungsgemäße Extrakt dadurch gekennzeichnet, dass er Flavone, Isoflavone, Chalkone und Pterocarpane enthält. Die Flavonverbindungen können isoprenyliert sein, wie z.B. Prenylderivate des Naringenins. Die Isoflavonverbindungen können ggf. mono-, di- oder trihydroxysubstitutiert oder O-methyliert sein. Weiterhin kann der Extrakt glykosidierte Flavon- und Isoflavonverbindungen enthalten. Der erfindungsgemäße Extrakt enthält insbesondere Maackiain, Maackiainglucosid, 8-Prenylnaringenin, Kushenol X, 8-Prenylkämpferol, Leachianon G, Kushenol E und 2,4,4',6'-Tetrahydroxy-3'-lavandulyl-2'-Methoxychalcon.

Der Extrakt kann aus Sophora-Arten ausgewählt aus der Gruppe bestehend aus *Sophora flavescens, Sophora subprostrata, Sophora alopeculoides, Sophora japonica, Sophora tonkinensis, Sophora tomentosa, Sophora moorcroftiana* und *Sophora leachiana,* vorzugsweise *Sophora flavescens* und *Sophora subprostrata* hergestellt werden.

Die erfindungsgemäß erhältlichen Extrakte können zusammen mit üblichen pharmazeutisch verträglichen Hilfsstoffen zu pharmazeutischen Zubereitungen wie Kapseln, Filmtabletten und Dragees verarbeitet werden, wobei als übliche pharmazeutisch verträgliche Hilfsstoffe Füll-, Binde-, Spreng-, Schmier- und Überzugsmittel für Filmtabletten und Dragees sowie Öle und Fette als Füllmassen für Kapseln verwendet werden können.

Die erfindungsgemäß erhältlichen Extrakte können zur Herstellung eines Medikaments zur Prophylaxe und Therapie von Krankheitszuständen verwendet werden, die durch einen Mangel an Östrogenen oder durch eine Dysregulation des Geschlechtshormonstoffwechsels, insbesondere Östrogenstoffwechsels verursacht werden.

Die Krankheitszustände können aus der Gruppe bestehend aus klimakterischen Beschwerden, geschlechtshormonabhängigen Krebserkrankungen, benigne Prostatahyperplasie, Osteoporose, Alzheimerische Krankheit und Herz-Kreislauferkrankungen ausgewählt sein, wobei die Krebserkrankungen Brustkrebs, Prostatakrebs und Gebärmutterkrebs umfassen.

Die erfindungsgemäßen Extrakte werden am Menschen in einer Dosierung von 1-1000 mg/täglich, vorzugsweise 100-1000 mg/täglich angewendet.

Mit einer erfindungsgemäßen Kombination umfassend Flavone, Isoflavone, Chalkone und Pterocarpane und weitere ähnliche Verbindungen wird somit ein Arzneimittel zur Verfügung gestellt, das zur Bekämpfung von Krankheitszuständen, die durch einen Mangel an Östrogen oder durch eine Dysregulation des Geschlechtshormonstoffwechsels, insbesondere Östrogenstoffwechsels verursacht werden, sehr gut geeignet ist.

Die nachstehend angegebenen Beispiele erläutern die Erfindung und sind nicht beschränkend aufzufassen. Alle Prozentangaben beziehen sich auf das Gewicht, falls nichts Anderes angegeben ist.

### Beispiel 1: Herstellung eines Extraktes aus Sophora flavescens

### a) Extraktion

2 kg gemahlene Wurzeln von *S. flavescens* wurden mit 14 kg 70 % (v/v) (62 % (w/w)) Ethanol versetzt, 5 min wirbelextrahiert (Ultraturrax) und 1 h bei 50°C intensiv gerührt. Anschließend wurde über ein Supra Seitz 1500-Filter abgesaugt und der Drogenrückstand in der gleichen Weise noch ein zweites Mal extrahiert. Die beiden Extraktlösungen wurden vereinigt und mit einem Aliquot wurde der Trockenextraktgehalt bestimmt. Es ergab sich ein Trockenrückstand von 475,6 g entsprechend einer Ausbeute von 23,8 %.

### b) Verteilung

Der Extraktionslösung wurde am Rotationsverdampfer bei 50°C das Ethanol entzogen. Der wässrige Rückstand (5 1) wurde 3x mit jeweils 2 l Ethylacetat ausgerührt, die Ethylacetat-Phasen vereinigt und am Rotationsverdampfer zur Trockne eingeengt:
Ethylacetat-Extrakt = 67,84 g (3,4 % bez. auf Droge; 14,3 % bez. auf Gesamtextrakt)

Der Ethylacetat-Extrakt enthält gemäß HPLC-Analyse Flavone, Isoflavone, Chalkone und Pterocarpane (z.B. 8-Prenylnaringenin, Daidzein, Kushenol X, Norkurarinon, Maackiain, Genistein, 8-Prenylkämpferol). Alkaloide konnten nicht nachgewiesen werden.

Diese Substanzkombination ist in idealer Weise zur Prophylaxe und Therapie von Krankheitszuständen geeignet, die durch einen Mangel an Östrogenen oder durch eine Dysregulation des Geschlechtshormonstoffwechsels, insbesondere Östrogenstoffwechsels verursacht werden (vgl. Beispiel 5).

### Beispiel 2: Herstellung eines Extraktes aus Sophora subprostrata

### a) Extraktion

1 kg gemahlene Wurzeln von *S. subprostrata* wurden mit 7 kg 70 % (v/v) (62 % (w/w)) Ethanol versetzt, 5 min wirbelextrahiert (Ultraturrax) und 1 h bei 50°C intensiv gerührt. Anschließend wurde über ein Supra Seitz 1500-Filter abgesaugt und der Drogenrückstand in der gleichen Weise noch ein zweites Mal extrahiert. Die beiden Extraktlösungen wurden vereinigt und mit einem Aliquot wurde der Trockenextraktgehalt bestimmt. Es ergab sich ein Trockenrückstand von 111,8 g entsprechend einer Ausbeute von 11,2 %.

### b) Verteilung

Der Extraktionslösung wurde am Rotationsverdampfer bei 50°C das Ethanol entzogen. Der wässrige Rückstand (2,5 1) wurde 3x mit jeweils 1 l Ethylacetat ausgerührt, die Ethylacetat-Phasen vereinigt und am Rotationsverdampfer zur Trockne eingeengt:
Ethylacetat-Extrakt = 10, 4 g (1,0 % bez. auf Droge; 9, 3 % bez. auf Gesamtextrakt)

### Beispiel 3

36 kg gemahlene *Sophora flavescens* Droge wurden mit der 7,7-fachen Gewichtsmenge 60 Gew.% Ethanol versetzt. Unter starkem Rühren und über Dispax (Ultraturrax) im Kreislauf wurde die Extraktlösung 1 h bei 50°C extrahiert. Die Exträktlösung wurde klarfiltriert, der Drogenrückstand noch einmal mit der 7-fachen Gewichtsmenge 60Gew.% Ethanol unter gleichen Bedingungen extrahiert und anschließend filtriert.

Die Extraktlösung wurde dann im Zentrifugalverdampfer auf einen Ethanol Gehalt von 3,5% und einen Trockenextraktgehalt von 10,52% eingeengt. Anschließend wurde diese konzentrierte Lösung 3 mal mit 1:0,4 Volumenteilen gegen wassergesättigten Ethylacetat verteilt. Die Ethylacetat-Phase wurde am Rotationsvedampfer eingeengt und im Trockenschrank bei 60°C nachgetrocknet.

Es resultierten:
Ethylacetat-Extrakt: 1,188 kg nach Vermahlung (3,3% bez. auf Droge)
HPLC-Gehalte pharmakologisch relevanter Inhaltsstoffe:

| | |
|---|---|
| Maackiainglucosid | 2,8 % |
| 8-Prenylnaringenin | 0,4 % |
| Maackiain | 0,6 % |
| Kushenol X | 1,3 % |
| 8-Prenylkämpferol | 0,7 % |
| Leachianon G | 6,6 % |
| Kushenol E | 0,3 % |
| 2,4,4',6'-Tetrahydroxy-3'-lavandulyl-2'-Methoxychalcon | 0,7 % |

Daidzein und Genistein konnten ebenfalls nachgewiesen werden. Alkaloide waren nicht nachweisbar.

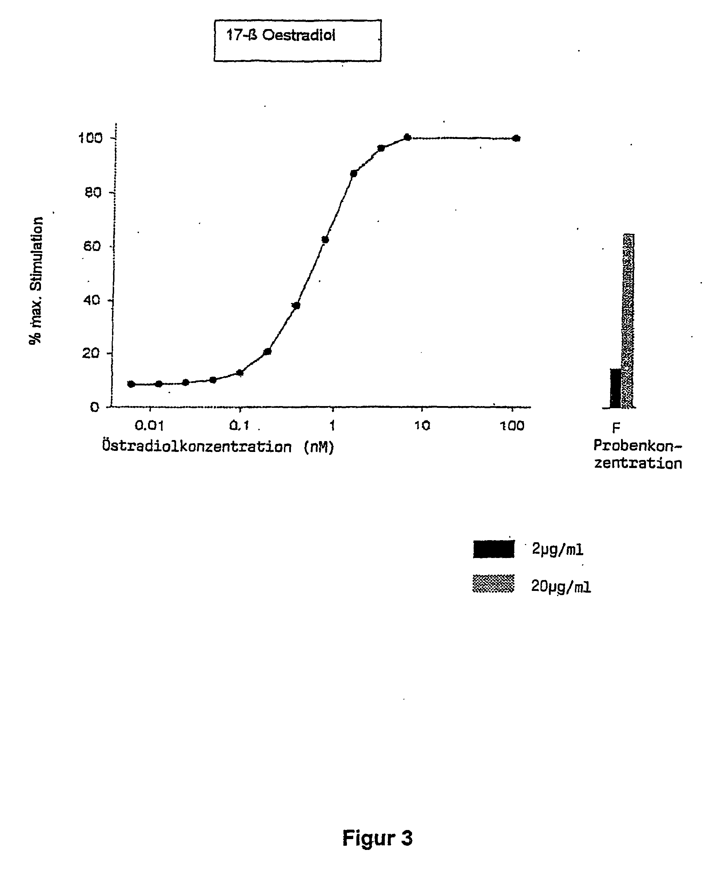

Die östrogene Aktivität konnte für jeden der angegebenen Inhaltsstoffe in einem Hefe-Assay entsprechend Beispiel 5 nachgewiesen werden. Für den Extrakt ergibt sich die östrogene Aktivität aus dem Reportergen-Assay unter Verwendung von Hefezellen gemäß Beispiel 5; vgl. Figur 3.

| HPLC-Methode | | |
|---|---|---|
| **Säule** | **LiChrospher 100 5 µm, 250 x 4 mm** | |
| Eluens | A: 1000 ml bidest Wasser / 3 ml Phosphorsäure (85%)/ 2 ml Triethylamin | |
| | B:100 ml Acetonitril/ 3 ml Phosphorsäure (85%)/ 2 ml Triethylamin /60 ml bidest Wasser | |
| Gradient | % A | % B |
| | 0 min 70 | 30 |
| | 30 min 30 | 70 |
| | 35 min 0 | 100 |
| Fluß | 1,2 ml/min | |
| Detektion | 220 nm | |

### Beispiel 4

150 g gemahlene *Sophora flavescens* Droge wurde zweimal mit wassergesättigtem Ethylacetat im Verhältnis 1:7 (m/m) extrahiert. Dazu wurde die Droge zuvor jeweils 5 Minuten mit einem Ultra-Turrax aufgeschlossen (Bewegungsextraktion). Die Droge wurde sodann je 1 h bei 60°C unter Rückfluss extrahiert. Die vereinigten Extraktlösungen wurden danach abfiltriert und die erhaltene Ethylacetat-Extraktlösung zweimal mit Ethylacetatgesättigtem Wasser im Verhältnis 1:1 (V/V) zurückgeschüttelt. Die vereinigten Ethylacetat-Phasen wurden zur Trockene eingeengt:
Ausbeute: 3,99% bez. auf die Droge

### Beispiel 5: Prüfung der hergestellten Ethylacetatextrakte auf östrogene Aktivität mit transfizierten Hefezellen, die den menschlichen α-Östrogenrezeptor exprimieren.

Die Prüfung von Extrakten auf östrogene Eigenschaften erfolgte mit einem Reportergen-Assay unter Verwendung von Hefezellen (Saccharomyces). Die Zellen sind stabil mit dem humanen α-Östrogenrezeptor und einem Expressionsplasmid, das ein Östrogenresponse-Element und das Gen für das Enzym β-Galaktosidase enthält, transfiziert. Alle Proben wurden in einer Konzentration von 20 mg/ml in DMSO gelöst und unverdünnt oder nach Verdünnen mit DMSO im Verhältnis 1/10, 1/100 oder in einem Volumen von 1 µl zu 100 µl Kulturmedium in 96-Well Flachboden-Mikrotiterplatten gegeben. Anschließend wurden 100 µl Hefesuspension und das chromogene Substrat Chlorphenolrot-β-D-Galactopyranosid zugefügt. Auf jeder Platte wurden zur Kontrolle Wells vorbereitet, in die nur Kulturmedium bzw. das Lösungsmittel eingefüllt wurde oder die die Standardkonzentrationen von 17β-Östradiol enthielten. Die Hefezellen wurden 72 h bei 32°C inkubiert und dann wurde die Absorption des Mediums bei 540 nm in einem Mikrotiterplatten-Photometer gemessen. Die Proben wurden teilweise zweimal geprüft.

### Ergebnisse:

| Probenkonzentrationen: | |
|---|---|
| **Verdünnung** | **Konzentration** |
| unverdünnt | 100 µg/ml |
| 1:10 | 10 µg/ml |
| 1:100 | 1 µg/ml |

| Probenbezeichnung: | | | |
|---|---|---|---|
| **Proben-Nr.** | **Probe** | **Versuch** | ***Sophora-*Art** |
| A | 70 % (v/v) (62 % (w/w)) Ethanol-Extrakt gemäß Beispiel 1a) | 1 | *Sophora flavescens* |
| B | 70 % (v/v) (62 % (w/w)) Ethanol-Extrakt gemäß Beispiel 2a) | 1 | *Sophora subprostrata* |
| C | 70 % (v/v) (62 % (w/w)) Ethanol-Extrakt gemäß Beispiel 1a) | 2 | *Sophora flavescens* |
| D | Ethylacetat-Extrakt gemäß Beispiel 1b) | 1 | *Sophora flavescens* |
| E | Ethylacetat-Extrakt gemäß Beispiel 2b) | 1 | *Sophora subprostrata* |
| F | Ethylacetat-Extrakt gemäß Beispiel 3 | 1 | *Sophora flavescens* |

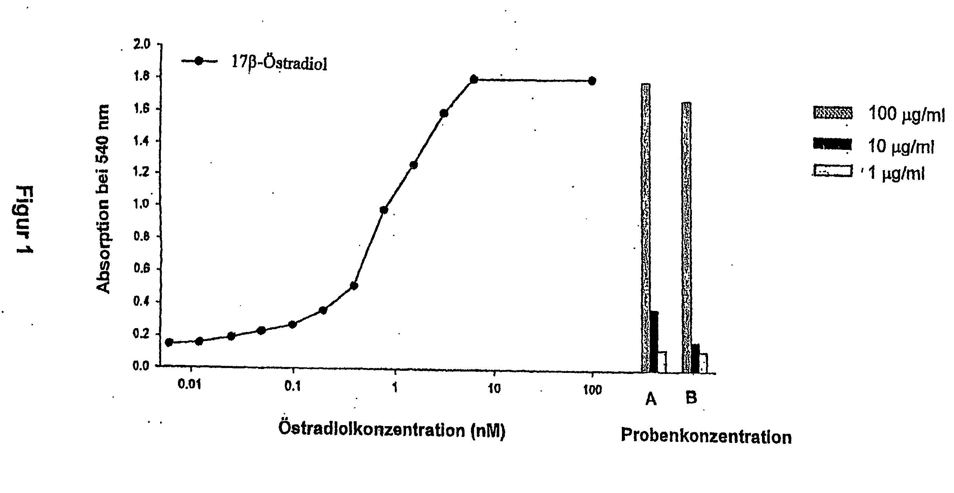

Die Ergebnisse des Assays sind in den Fig. 1, 2 und 3 dargestellt. Hierbei wird die östrogene Aktivität der Extrakte mit der Aktivität von 17β-Östradiol verglichen. Als "aktiv" können hierbei jene Extrakte bezeichnet werden, deren Aktivität beim Vergleich mit 17β-Östradiol einer 17β-Östradiol-Aktivität entspricht, die innerhalb des deutlich ansteigenden Abschnitts (also ab einer Konzentration von etwa 0,1-0,2 nM 17β-Östradiol) der Aktivitätskurve des 17β-Östradiol angesiedelt ist.

Insbesondere beschreibt Figur 1 die östrogene Aktivität von 70 % (v/v) (62 % (w/w)) Ethanol-Extrakten (gemäß der Beispiele 1a) und 2a)) aus *Sophora flavescens* und *Sophora subprostrata* in einem Hefe-Assay, verglichen mit 17β-Östradiol.

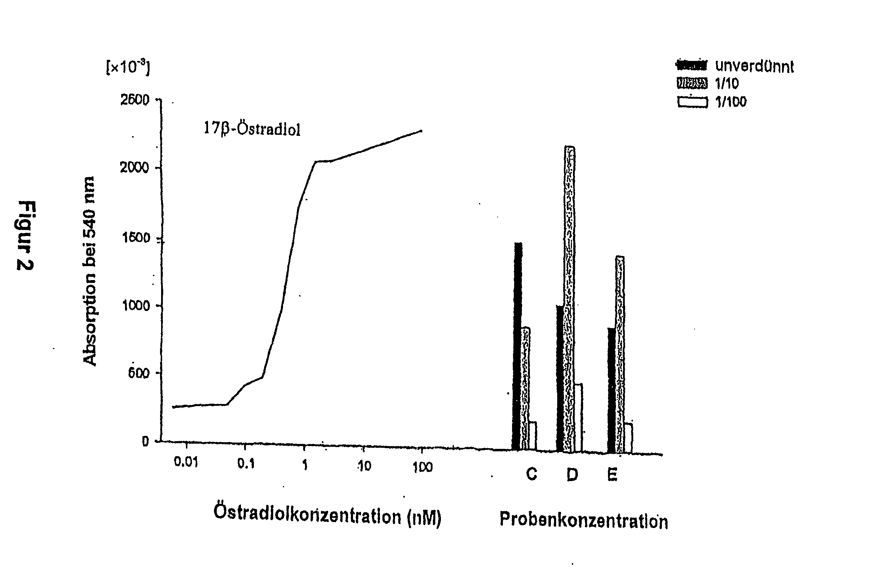

Figur 2 beschreibt die östrogene Aktivität von erfindungsgemäßen Extrakten (70 % (v/v) bzw. 62 % (w/w) Ethanol-Extrakte nach Verteilung mit Ethylacetat; gemäß der Beispiele 1b) und 2b)) aus *Sophora flavescens* und *Sophora subprostrata* in einem Hefe-Assay, verglichen mit 17β-Östradiol.

Figur 3 beschreibt die östrogene Aktivität von erfindungsgemäßen Extrakten (60 % (w/w) Ethanol-Extrakte nach Verteilung mit Ethylacetat gemäß Beispiel 3) aus *Sophora flavescens* in einem Hefe-Assay, verglichen mit 17β-Östradiol.

## Patentansprüche

1. Extrakt aus Sophora-Arten, **dadurch gekennzeichnet, dass** er nicht mehr als 0,2 % Gesamtalkaloide enthält, und dass er Flavone, Isoflavone, Chalkone und Pterocarpane enthält, wobei die Flavone 8-Prenylnaringenin, Kushenol X, 8-Prenylkämpferol, Leachianon G und Kushenol E umfassen, wobei die Isoflavone Daidzein und Genistein umfassen, wobei die Chalkone 2,4,4',6'-Tetrahydroxy-3'-lavandulyl-2'-Methoxychalcon umfassen und wobei die Pterocarpane Maackiain und Maackiainglucosid umfassen.

2. Extrakt nach Anspruch 1, **dadurch gekennzeichnet, dass** er nicht mehr als 0,1 % Gesamtalkaloide enthält.

3. Extrakt nach Anspruch 1, **dadurch gekennzeichnet, dass** er alkaloidfrei ist.

4. Extrakt nach einem der Ansprüche 1 bis 3, wobei die *Sophora*-Art ausgewählt ist aus der Gruppe bestehend aus *Sophora flavescens, Sophora subprostrata, Sophora alopeculoides, Sophora japonica, Sophora tonkinensis, Sophora tomentosa, Sophora moorcroftiana* und *Sophora leachiana*.

5. Extrakt nach einem der Ansprüche 1 bis 3, wobei die *Sophora*-Art ausgewählt ist aus *Sophora flavescens* und *Sophora subprostrata*.

6. Verfahren zur Gewinnung eines Extraktes aus *Sophora*-Arten, **gekennzeichnet durch** die Schritte:
a) ein- oder mehrmaliges Extrahieren von Sophora-Arten mit einem Lösungsmittel ausgewählt aus der Gruppe bestehend aus
i) wässrigen Alkoholen und wässrigen Ketonen; und
ii) Estern einschließlich wässrigen oder wassergesättigten Estern;
b) Abziehen des Lösungsmittels von der in Schritt a)i) erhaltenen alkoholischen oder ketonischen Extraktionslösung;
c)i) ein- oder mehrmalige Verteilung des in Schritt b) erhaltenen Rückstands zwischen einem organischen Lösungsmittel ausgewählt aus der Gruppe bestehend aus Ethylacetat, tert-Butylmethylether, n-Butanol und Butanon und Wasser und Vereinigen der erhaltenen Lösungsmittelphasen; oder
c)ii) ein- oder mehrmalige direkte Verteilung des in Schritt a)ii) erhaltenen Extrakts mit Wasser; und
d) Einengen der in c) erhaltenen vereinigten organischen Lösungsmittelphasen zur Trockne.

7. Verfahren nach Anspruch 6, wobei die Extraktion in Schritt a) zweimal ausgeführt wird.

8. Verfahren nach Anspruch 6 oder 7, wobei das in Schritt a)i) verwendete Lösungsmittel wässriges Ethanol ist.

9. Verfahren nach Anspruch 6 oder 7, wobei das in Schritt a)i) verwendete Lösungsmittel wässriges Aceton ist.

10. Verfahren nach einem der Ansprüche 6 bis 9, wobei der in Schritt a)i) verwendete Alkohol als 10-96 % (v/v) oder (w/w) und das in Schritt a)i) verwendete Keton als 10-99 % (v/v) oder (w/w) wässriges Gemisch vorliegt.

11. Verfahren nach einem der Ansprüche 6 bis 9, wobei der in Schritt a)i) verwendete Alkohol oder das in Schritt a)i) verwendete Keton als 50-92 % (v/v) oder (w/w) wässriges Gemisch vorliegt.

12. Verfahren nach Anspruch 6 oder 7, wobei das in Schritt a)i) verwendete Lösungsmittel 70 % (v/v) oder 60 % (w/w) wässriges Ethanol ist.

13. Verfahren nach Anspruch 6 oder 7, wobei das in Schritt a)ii) verwendete Lösungsmittel Ethylacetat oder wässriges oder wassergesättigtes Ethylacetat ist.

14. Verfahren nach einem der Ansprüche 6 bis 13, wobei das in Schritt c)i) verwendete Lösungsmittel Ethylacetat ist.

15. Verfahren nach einem der Ansprüche 6 bis 14, wobei die *Sophora*-Art ausgewählt ist aus der Gruppe bestehend aus *Sophora flavescens, Sophora subprostrata, Sophora alopeculoides, Sophora japonica, Sophora tonkinensis, Sophora tomentosa, Sophora moorcroftiana* und *Sophora leachiana*.

16. Verfahren nach einem der Ansprüche 6 bis 14, wobei die *Sophora*-Art ausgewählt ist aus *Sophora flavescens* und *Sophora subprostrata*.

17. Pharmazeutische Zubereitung umfassend einen Extrakt nach einem der Ansprüche 1 bis 5 und übliche pharmazeutisch verträgliche Hilfsstoffe.

18. Verwendung eines Extrakts nach einem der Ansprüche 1 bis 5 oder einer pharmazeutischen Zubereitung nach Anspruch 17 zur Herstellung eines Medikaments zur Prophylaxe und Therapie von Krankheitszuständen, die durch einen Mangel an Östrogenen oder durch eine Dysregulation des Geschlechtshormonstoffwechsels, insbesondere Östrogenstoffwechsels verursacht werden.

19. Verwendung nach Anspruch 18, wobei die Krankheitszustände aus der Gruppe bestehend aus klimakterischen Beschwerden, geschlechtshormonabhängigen Krebserkrankungen, benigner Prostatahyperplasie, Osteoporose, Alzheimerscher Krankheit und Herz-Kreislauferkrankungen ausgewählt sind.

20. Verwendung nach Anspruch 19, wobei die geschlechtshormonabhängigen Krebserkrankungen aus der Gruppe bestehend aus Brustkrebs, Prostatakrebs und Gebärmutterkrebs ausgewählt sind.

## Claims

1. Extract from *Sophora* species, **characterised in that** it contains not more than 0.2% alkaloids in total, and that it contains flavones, isoflavones, chalcones and pterocarpanes, wherein the flavones include 8-prenylnaringenine, kushenol X, 8-prenylkaempferol, leachianon G and kushenol E, wherein the isoflavones include daidzein and genistein, wherein the chalcones include 2,4,4',6'-tetrahydroxy-3'-lavandulyl-2'-methoxychalcone, and wherein the pterocarpanes include maackiain and maackiain-glucoside.

2. Extract according to claim 1, **characterised in that** it contains not more than 0.1% alkaloids in total.

3. Extract according to claim 1, **characterised in that** it is free of alkaloids.

4. Extract according to any of claims 1 to 3, wherein the species of *Sophora* is selected from the group consisting of *Sophora flavescens, Sophora subprostrata, Sophora alopeculoides, Sophora japonica, Sophora tonkinensis, Sophora tomentosa, Sophora moorcroftiana* and *Sophora leachiana*.

5. Extract according to any of claims 1 to 3, wherein the species of *Sophora* is selected from *Sophora flavescens* and *Sophora subprostrata*.

6. Method for obtaining an extract from Sophora species, **characterized by** the steps:
a) one or more extractions of Sophora species with a solvent selected from the group consisting of
i) aqueous alcohols and aqueous ketones; and
ii) esters, including aqueous or water-saturated esters;
b) draining off the solvent from the alcoholic or ketonic extraction solution obtained at step a)i);
c) i) one or more distributions of the residue obtained at step b) between an organic solvent selected from the group consisting of ethylacetate, tert-butylmethylether, n-butanol and butanon and water, and combining the solvent phases thus obtained; or
c) ii) one or more direct distributions of the extract obtained at step a)ii) with water; and
d) concentrating the combined organic solvent phases obtained in c) till dry.

7. Method according to claim 6, wherein extraction at step
a) is performed twice.

8. Method according to claim 6 or 7, wherein the solvent used at step a)i) is aqueous ethanol.

9. Method according to claim 6 or 7, wherein the solvent used at step a)i) is aqueous acetone.

10. Method according to any of claims 6 to 9, wherein the alcohol used at step a)i) is present as 10-96% [v/v] or [w/w] or the ketone used at step a)i) is present as 10-99% [v/v] or [w/w] aqueous mixture.

11. Method according to any of claims 6 to 9, wherein the alcohol used at step a)i) or the ketone used at step a)i) is present as 50-92% [v/v] or [w/w] aqueous mixture.

12. Method according to claim 6 or 7, wherein the solvent used at step a)i) is 70% [v/v] or 60% [w/w] aqueous ethanol.

13. Method according to claim 6 or 7, wherein the solvent used at step a)ii) is ethylacetate or aqueous or water-saturated ethylacetate.

14. Method according to any of claims 6 to 13, wherein the solvent used at step c)i) is ethylacetate.

15. Method according to any of claims 6 to 14, wherein the species of *Sophora* is selected from the group consisting of *Sophora flavescens, Sophora subprostrata*, *Sophora alopeculoides, Sophora japonica, Sophora tonkinensis, Sophora tomentosa, Sophora moorcroftiana* and *Sophora leachiana*.

16. Method according to any of claims 6 to 14, wherein the species of *Sophora* is selected from *Sophora flavescens* and *Sophora subprostrata*.

17. Pharmaceutical preparation including an extract according to any of claims 1 to 5 and conventional pharmaceutically-acceptable additives.

18. Use of an extract according to any of claims 1 to 5, or a pharmaceutical preparation according to claim 17, for the preparation of a medicament for the prophylaxis and treatment of pathological conditions caused by a deficiency of oestrogens or by a dysregulation of sex hormone metabolism, particularly oestrogen metabolism.

19. Use according to claim 18, wherein the pathological condition is selected from the group consisting of climacteric complaints, sex hormone-dependent cancers, benign prostate hyperplasia, osteoporosis, Alzheimer's disease and cardiovascular diseases.

20. Use according to claim 19, wherein the sex hormone-dependent cancers are selected from the group consisting of breast cancer, prostate cancer and cancer of the womb.

## Revendications

1. Extrait à base d'espèces de Sophora, **caractérisé en ce qu'**il ne contient pas plus de 0,2 % d'alcaloïdes totaux et **en ce qu'**il contient des flavones, des isoflavones, des chalcones et des ptérocarpanes, les flavones comprenant la 8-prénylnaringénine, le kushénol X, le kaempferol 8-prénylée, la léachianone G et le kushénol E, les isoflavones comprenant la daidzéine et la génistéine, les chalcones comprenant la 2,4,4',6'-tétrahydroxy-3'-lavandulyl-2'-méthoxychalcone, et les ptérocarpanes comprenant la maackiaïne et le glucoside de maackiaïne.

2. Extrait selon la revendication 1, **caractérisé en ce qu'**il ne contient pas plus de 0,1 % d'alcaloïdes totaux.

3. Extrait selon la revendication 1, **caractérisé en ce qu'**il est exempt d'alcaloïdes.

4. Extrait selon l'une des revendications 1 à 3, l'espèce de *Sophora* étant choisie dans l'ensemble constitué de *Sophora flavescens, Sophora subprostrata, Sophora alopeculoides, Sophora japonica, Sophora tonkinensis, Sophora tomentosa, Sophora moorcroftiana* et *Sophora leachiana*.

5. Extrait selon l'une des revendications 1 à 3, l'espèce de *Sophora* étant choisie parmi *Sophora flavescens* et *Sophora subprostrata*.

6. Procédé de préparation d'un extrait à base d'espèces de Sophora, **caractérisé par** les étapes consistant à:
a) extraire une ou plusieurs fois des espèces de Sophora avec un solvant choisi dans l'ensemble constitué de:
i) des alcools aqueux et des cétones aqueuses ; et
ii) des esters, y compris des esters aqueux ou saturés d'eau ;
b) éliminer le solvant de la solution d'extraction alcoolique ou cétonique obtenue dans l'étape a)i) ;
c)i) répartir une ou plusieurs fois le résidu obtenu dans l'étape b) entre un solvant organique choisi dans l'ensemble constitué de l'acétate d'éthyle, l'éther tert-butylméthylique, le n-butanol et la butanone, et de l'eau, et combiner les phases de solvant obtenues ; ou
c)ii) répartir directement, une ou plusieurs fois, l'extrait obtenu dans l'étape a)ii) avec de l'eau ; et
d) concentrer par évaporation les phases de solvant organique combinées obtenues en c) jusqu'à siccité.

7. Procédé selon la revendication 6, dans lequel on effectue deux fois l'extraction dans l'étape a).

8. Procédé selon la revendication 6 ou 7, dans lequel le solvant utilisé dans l'étape a)i) est de l'éthanol aqueux.

9. Procédé selon la revendication 6 ou 7, dans lequel le solvant utilisé dans l'étape a)i) est de l'acétone aqueuse.

10. Procédé selon l'une des revendications 6 à 9, dans lequel l'alcool utilisé dans l'étape a)i) se présente sous la forme d'un mélange aqueux à 10-96 % (v/v) ou (p/p), et la cétone utilisée dans l'étape a)i) se présente sous la forme d'un mélange aqueux à 10-99 % (v/v) ou (p/p).

11. Procédé selon l'une des revendications 6 à 9, dans lequel l'alcool utilisé dans l'étape a)i) ou la cétone utilisée dans l'étape a)i) se présente sous la forme d'un mélange aqueux à 50-92 % (v/v) ou (p/p).

12. Procédé selon la revendication 6 ou 7, dans lequel le solvant utilisé dans l'étape a)i) est de l'éthanol aqueux à 70 % (v/v) ou à 60 % (p/p).

13. Procédé selon la revendication 6 ou 7, dans lequel le solvant utilisé dans l'étape a)ii) est de l'acétate d'éthyle ou de l'acétate d'éthyle aqueux ou saturé d'eau.

14. Procédé selon l'une des revendications 6 à 13, dans lequel le solvant utilisé dans l'étape c)i) est de l'acétate d'éthyle.

15. Procédé selon l'une des revendications 6 à 14, dans lequel l'espèce de Sophora est choisie dans l'ensemble constitué de *Sophora flavescens, Sophora subprostrata, Sophora alopeculoides, Sophora japonica, Sophora tonkinensis, Sophora tomentosa, Sophora moorcroftiana* et *Sophora leachiana*.

16. Procédé selon l'une des revendications 6 à 14, dans lequel l'espèce de Sophora est choisie parmi *Sophora flavescens* et *Sophora subprostrata*.

17. Préparation pharmaceutique comprenant un extrait selon l'une des revendications 1 à 5 et des adjuvants usuels pharmaceutiquement acceptables.

18. Utilisation d'un extrait selon l'une des revendications 1 à 5 ou d'une préparation pharmaceutique selon la revendication 17 pour la fabrication d'un médicament pour la prophylaxie et le traitement d'états maladifs qui sont provoqués par une carence en oestrogènes ou par un dérèglement du métabolisme des hormones sexuelles, en particulier du métabolisme des oestrogènes.

19. Utilisation selon la revendication 18, dans laquelle les états maladifs sont choisis dans l'ensemble consistant en les douleurs climatériques, les maladies cancéreuses dépendantes des hormones sexuelles, l'hyperplasie bénigne de la prostate, l'ostéoporose, la maladie d'Alzheimer et les maladies de la circulation cardiaque.

20. Utilisation selon la revendication 19, dans laquelle les maladies cancéreuses dépendantes des hormones sexuelles sont choisies dans l'ensemble consistant en le cancer du sein, le cancer de la prostate et le cancer de l'utérus.
